# EUROPEAN PATENT APPLICATION

(11) **EP 3 309 747 A1**
(43) Date of publication of application: **18.04.2018**
(21) Application number: 16807455.7
(22) Date of filing: 07.06.2016
(51) Int. Cl.: G06T 7/20, A61B 5/00, A61G 7/05, G06T 1/00, G08B 25/00, G08B 25/04

(54) **MOTION DETECTION SYSTEM, MOTION DETECTION DEVICE, MOTION DETECTION METHOD, AND MOTION DETECTION PROGRAM**

(30) Priority: 11.06.2015 JP 2015118048
(71) Applicant: Konica Minolta, Inc., Tokyo 100-7015 (JP)
(72) Inventor: HORITA, Shinichi, Tokyo 100-7015 (JP)
(74) Representative: Gille Hrabal
(86) International application number: PCT/JP2016/066855
(87) International publication number: WO 2016/199748

(57) **Abstract**

Provided is a motion detection system capable of alleviating a burden on a caregiver which could arise when a plurality of types of notification target motions is detected during a short time period. A motion detection system (300) includes: an acquisition unit (52) for acquiring motion data indicating a motion of a person; a detection unit (150) for detecting a predetermined motion of the person using the motion data; a selection unit (160) for, when a plurality of types of output candidate motions is detected by the detection unit (150) during a predetermined time period, selecting an output target motion from among the output candidate motions such that types of the output candidate motions are made fewer than the plurality of types; and an output unit (170) for outputting the output target motion selected by the selection unit (160).

## Description

### Technical Field

The present disclosure relates to a motion detection system, a motion detection device, a motion detection method, and a motion detection program capable of judging a motion of a person.

### Background Art

There is a technology for judging a motion of a person from an image. This technology is applied to, for example, a motion detection system that watches motions of care receivers such as elderly people and patients who need nursing care. This motion detection system detects that a care receiver has performed a dangerous motion that may bring about falling or the like (for example, rising on bed or leaving bed) and notifies a caregiver of this fact. As a result, the caregiver can rush to the care receiver and thus can prevent the care receiver from, for example, falling beforehand.

Regarding such a motion detection system, JP 2009-5963 A (Patent Literature 1) discloses a pre-leaving motion detection device that "prevents detection of a pre-leaving motion of a patient when someone is present near the patient". JP 2001-327549 A (Patent Literature 2) discloses a dynamic state detection system that "detects a series of motions from a sleeping posture to the start of walking of a care receiver by dynamic state".

### Citation List

### Patent Literature

Patent Literature 1: JP 2009-5963 A
Patent Literature 2: JP 2001-327549 A

### Summary of Invention

### Technical Problem

A motion detection system sometimes detects a plurality of types of motions during a short time period. When a motion of a care receiver is notified to a caregiver every time the motion is detected, a burden on the caregiver increases. Therefore, a motion detection system capable of alleviating a burden on the caregiver caused by this situation is desired.

When a contact of a patient's hand or the like is sensed and a distance output by a distance sensor is within a range settled in advance, the pre-leaving motion detection device disclosed in Patent Literature 1 does not notify a pre-leaving motion of the patient. That is, this pre-leaving motion detection device merely determines whether to notify that the patient has left the bed and does not detect a plurality of types of motions.

The dynamic state detection system disclosed in Patent Literature 2 detects a series of dynamic states from a sleeping posture to a long sitting position, a chair sitting position, a standing position, and the start of walking of a care receiver gradually for each dynamic state. This dynamic state detection system detects a sequence of the dynamic states in a case where a subsequent dynamic state is consecutively detected after a given time period elapsed since one of the dynamic states was detected. That is, this dynamic state detection system merely determines whether the dynamic states are continuing and not assumes a case where a plurality of types of dynamic states is detected within a given time period.

The present disclosure has been made to solve the above-described disadvantages and an object of a certain aspect is to provide a motion detection system capable of alleviating a burden on a caregiver which could arise when a plurality of types of notification target motions is detected during a short time period. An object of another aspect is to provide a motion detection device capable of alleviating a burden on a caregiver which could arise when a plurality of types of notification target motions is detected during a short time period. An object of a still another aspect is to provide a motion detection method capable of alleviating a burden on a caregiver which could arise when a plurality of types of notification target motions is detected during a short time period. An object of a still another aspect is to provide a motion detection program capable of alleviating a burden on a caregiver which could arise when a plurality of types of notification target motions is detected during a short time period.

### Solution to Problem

According to one aspect, a motion detection system includes: an acquisition unit for acquiring motion data indicating a motion of a person; a detection unit for detecting a predetermined motion of the person using the motion data; a selection unit for, when a plurality of types of output candidate motions is detected by the detection unit during a predetermined time period, selecting an output target motion from among the output candidate motions such that types of the output candidate motions are made fewer than the plurality of types; and an output unit for outputting the output target motion selected by the selection unit.

Preferably, the plurality of types of the output candidate motions includes a group of motions that could arise during an interval from when the person is sleeping in a bed to when leaving the bed.

Preferably, the plurality of types of the output candidate motions is a group of a series of motions that could arise in order of time series.

Preferably, the selection unit selects a last detected motion among the output candidate motions as the output target motion.

Preferably, the selection unit selects a first detected motion among the output candidate motions as the output target motion.

Preferably, a priority is associated in advance with each of target motions to be detected by the detection unit. The selection unit selects a motion having a highest priority among the output candidate motions as the output target motion.

Preferably, the group of the series of the motions includes a first motion, a second motion, and a third motion. The first motion and the second motion are motions that could arise before the third motion. When the third motion is detected, the selection unit changes the predetermined time period according to whether a motion detected before the third motion is the first motion or the second motion.

Preferably, the output unit outputs the output target motions by a plurality of different output means.
The selection unit selects one of the output candidate motions using the predetermined time period different for each of the plurality of output means.

Preferably, the output unit includes, as the plurality of output means, a first output means for notifying a caregiver for the person of the output target motion and a second output means for saving the output target motion to a memory. The selection unit selects one of the output candidate motions for the first output means at every first predetermined time period and selects one of the output candidate motions for the second output means at every second predetermined time period shorter than the first predetermined time period.

Preferably, the output unit includes, as the plurality of output means, a first output means for notifying a caregiver for the person of the output target motion and a second output means for notifying an administrator of the motion detection system of the output target motion. The selection unit selects one of the output candidate motions for the first output means at every first predetermined time period and selects one of the output candidate motions for the second output means at every second predetermined time period shorter than the first predetermined time period.

According to another aspect, a motion detection device includes: an acquisition unit for acquiring motion data indicating a motion of a person; a detection unit for detecting a predetermined motion of the person using the motion data; a selection unit for, when a plurality of types of output candidate motions is detected by the detection unit during a predetermined time period, selecting an output target motion from among the output candidate motions such that types of the output candidate motions are made fewer than the plurality of types; and an output unit for outputting the output target motion selected by the selection unit.

According to still another aspect, a motion detection method includes: a step of acquiring motion data indicating a motion of a person; a step of detecting a predetermined motion of the person using the motion data; a step of, when a plurality of types of output candidate motions is detected in the step of detecting during a predetermined time period, selecting an output target motion from among the output candidate motions such that types of the output candidate motions are made fewer than the plurality of types; and a step of outputting the output target motion selected in the step of selecting.

According to still another aspect, a motion detection program causes a computer to execute: a step of acquiring motion data indicating a motion of a person; a step of detecting a predetermined motion of the person using the motion data; a step of, when a plurality of types of output candidate motions is detected in the step of detecting during a predetermined time period, selecting an output target motion from among the output candidate motions such that types of the output candidate motions are made fewer than the plurality of types; and a step of outputting the output target motion selected in the step of selecting.

### Advantageous Effects of Invention

In one aspect, a burden on a caregiver which could arise when a plurality of types of notification target motions is detected during a short time period can be alleviated.

The above-described and other objects, features, aspects and advantages of the present invention will become apparent from the following detailed description regarding the present invention, which will be understood in conjunction with the accompanying drawings.

### Brief Description of Drawings

Fig. 1 is a diagram illustrating an example of the configuration of a motion detection system according to a first embodiment.
Fig. 2 is a block diagram illustrating a hardware configuration and a functional configuration of the motion detection system according to the first embodiment.
Fig. 3 is a diagram illustrating the contents of a motion set in the first embodiment.
Fig. 4 is a diagram illustrating an output method for a detected motion.
Fig. 5 is a flowchart illustrating a specific example 1-1 of selection processing.
Fig. 6 is a flowchart illustrating a specific example 1-2 of the selection processing.
Fig. 7 is a flowchart illustrating a specific example 1-3 of the selection processing.
Fig. 8 is a diagram illustrating the contents of a priority table.
Fig. 9 is a flowchart illustrating a specific example 1-4 of the selection processing.
Fig. 10 is a flowchart illustrating a specific example 1-5 of the selection processing.
Fig. 11 is a block diagram illustrating a main hardware configuration of the motion detection system according to the first embodiment.
Fig. 12 is a block diagram illustrating a hardware configuration and a functional configuration of a motion detection system according to a second embodiment.
Fig. 13 is a flowchart illustrating a specific example 2 of the selection processing.
Fig. 14 is a diagram illustrating the contents of a motion set in the second embodiment.
Fig. 15 is a block diagram illustrating a hardware configuration and a functional configuration of a motion detection system according to a third embodiment.
Fig. 16 is a flowchart illustrating a specific example 3 of the selection processing.

### Description of Embodiments

Hereinafter, each embodiment according to the present invention will be described with reference to the drawings. In the following description, the same members and constituent elements are denoted by the same reference numerals. The names and functions thereof are also the same. Therefore, detailed description thereof will not be repeated. Note that the respective embodiments and modifications described below may be selectively combined as appropriate.

### <First Embodiment

### [Device Configuration of Motion Detection System 300]

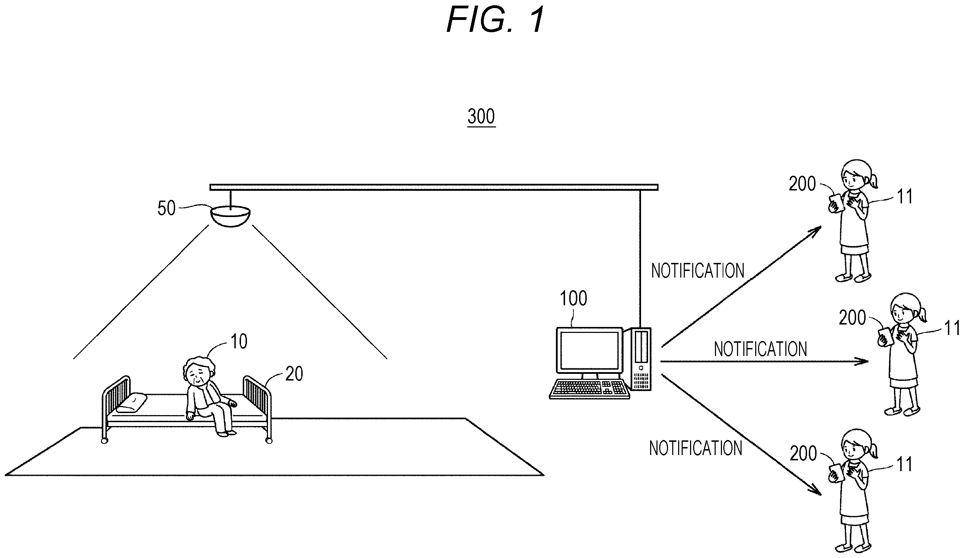

A device configuration of a motion detection system 300 will be described with reference to Fig. 1. Fig. 1 is a diagram illustrating an example of the configuration of the motion detection system 300.

The motion detection system 300 is used, for example, to watch a care receiver 10 who is a person to be monitored. As illustrated in Fig. 1, the motion detection system 300 includes a camera 50, a motion detection device 100, and mobile terminals 200. The camera 50 and the motion detection device 100 are connected to each other via a network. The motion detection device 100 and each mobile terminal 200 are connected to each other via a network.

The camera 50 is installed in, for example, a nursing facility, a medical facility, or a house. Fig. 1 illustrates how the camera 50 captures the care receiver 10 and a bed 20 from a ceiling. The camera 50 may be attached to the ceiling or attached to a side wall.

The motion detection device 100 judges a motion of the care receiver 10 based on a time-series image (video) obtained from the camera 50. As an example, motions of the care receiver 10 detectable by the motion detection device 100 include rising on bed and falling down from the bed 20 of the care receiver 10.

The motion detection device 100 regards a detected motion as a candidate of a motion to be notified to a caregiver 11 (hereinafter also referred to as "notification candidate"). When a plurality of types of notification candidate motions are detected during a short time period (for example, several seconds), the motion detection device 100 selects a notification target motion from among the notification candidate motions such that the types of motions to be notified are made fewer. That is, the motion detection device 100 selects a part of the notification candidate motions as a notification target. The motion detection device 100 transmits information indicating the type of the selected notification target motion to the mobile terminal 200 for the caregiver 11.

Upon receiving the information indicating the type of the notification target motion from the motion detection device 100, the mobile terminal 200 notifies the caregiver 11 that the notification target motion has been detected. As examples of a notification method, the mobile terminal 200 displays information indicating the type of motion as a message or issues the type of motion as a sound. As a result, the caregiver 11 can grasp the rising on bed, falling, and so on of the care receiver 10 and thus can quickly rush to the care receiver 10.

As described above, the motion detection device 100 selects a notification target motion from among the notification candidate motions and notifies the caregiver 11 of the notification target motion. As a result, the number of times of notifications to the caregiver 11 is reduced and a burden on the caregiver 11 is alleviated. In addition, since a minimum necessary motion of the care receiver 10 is notified to the caregiver 11, the caregiver 11 can easily grasp the state of the care receiver 10.

Note that, although Fig. 1 illustrates an example in which the motion detection system 300 is provided with one camera 50, the motion detection system 300 may be provided with a plurality of cameras 50. In addition, although Fig. 1 illustrates an example in which the motion detection system 300 is provided with one motion detection device 100, the motion detection system 300 may be provided with a plurality of motion detection devices 100. Furthermore, although the camera 50 and the motion detection device 100 are configured as separate devices in Fig. 1, the camera 50 and the motion detection device 100 may be configured integrally. Additionally, although Fig. 1 illustrates an example in which the motion detection system 300 is provided with a plurality of mobile terminals 200, the motion detection system 300 may be configured with one mobile terminal 200.

### [Processing by Motion Detection System 300]

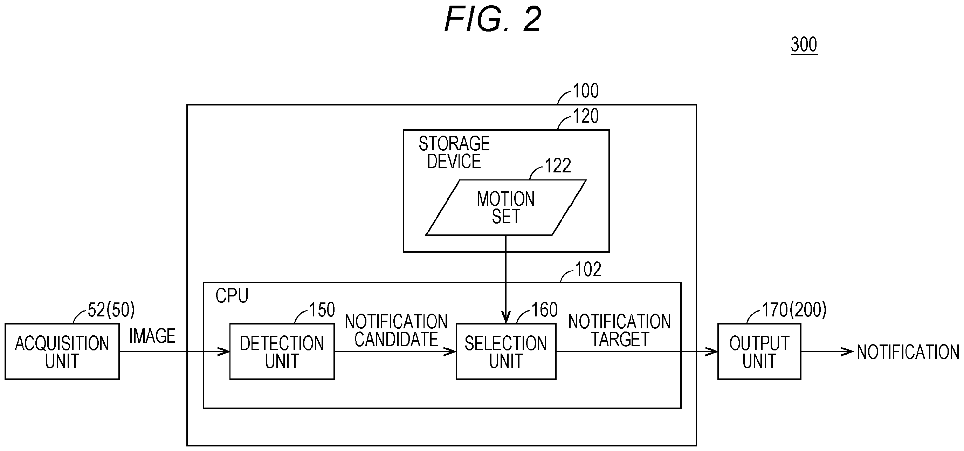

Motion detection processing by the motion detection system 300 according to the first embodiment will be described with reference to Fig. 2. Fig. 2 is a block diagram illustrating a hardware configuration and a functional configuration of the motion detection system 300 according to the first embodiment. As illustrated in Fig. 2, the motion detection system 300 is provided with an acquisition unit 52, the motion detection device 100, and an output unit 170. The motion detection device 100 includes a central processing unit (CPU) 102 and a storage device 120 as a hardware configuration. The CPU 102 has a detection unit 150 and a selection unit 160 as a functional configuration. Hereinafter, the acquisition unit 52, the detection unit 150, a motion set 122, the selection unit 160, and the output unit 170 will be described in order.

### (Acquisition Unit 52)

The acquisition unit 52 acquires motion data indicating a motion of the care receiver. The acquisition unit 52 is, for example, the camera 50 in Fig. 1. The acquisition unit 52 serving as the camera 50 acquires, as the motion data, an image obtained by capturing a person to be monitored. The image may be a still image or a moving image. The acquisition unit 52 sequentially outputs the acquired images to the detection unit 150.

Note that the acquisition unit 52 is not limited to the camera 50 as long as the acquisition unit 52 is a device capable of acquiring the motion amount of the care receiver. For example, the acquisition unit 52 may be a pressure sensitive mat disposed under the bed, a distance image sensor of a time of flight (TOF) technique, or the like. The pressure sensitive mat outputs a pressure value as the motion data. The distance image sensor outputs a distance image as the motion data. In addition, the acquisition unit 52 does not need to be constituted by one sensor and may be constituted by a plurality of sensors.

### (Detection Unit 150)

The detection unit 150 analyzes the image obtained from the acquisition unit 52 and detects a particular action of a caregiver. Motions detectable by the detection unit 150 include a group of motions that could arise during an interval from when the care receiver is sleeping in the bed to when leaving the bed. As mentioned above, by detecting the group of motions at the time of rising on bed, which is likely to lead to falling of the care receiver, the caregiver can more reliably prevent the care receiver from falling.

This group of motions includes, for example, lying in bed, rising on bed, chair sitting on bed end, leaving bed, and falling down. Lying in bed refers to a state in which the care receiver lies in bed. Rising on bed refers to a motion of the care receiver rising on the bed. Chair sitting on bed end refers to a state in which the care receiver is sitting on the bed or a state in which the body of the care receiver hangs on an edge of the bed. Leaving bed refers to a state in which the care receiver is away from the bed. Falling down refers to a state in which the care receiver drops out of the bed and lies on the floor.

As an example, the detection unit 150 calculates an evaluation value indicating the degree of movement of the care receiver using the motion data and also specifies a motion area within an image in which the movement of the care receiver is arising. When this evaluation value is smaller than a value settled in advance and the motion area is included in a bed area within the image, the detection unit 150 detects the motion "lying in bed". When this evaluation value is larger than a predetermined threshold value and the motion area is included in the bed area within the image, the detection unit 150 detects the motion "rising on bed". When the motion area overlaps with a bed boundary within the image, the detection unit 150 detects the motion "chair sitting on bed end". When this evaluation value is larger than the predetermined threshold value and the motion area is not included in the bed area within the image, the detection unit 150 detects the motion "leaving bed". When this evaluation value is larger than the predetermined threshold value and the motion area moves from the bed boundary to the outside of the bed, the detection unit 150 detects the motion "falling down". The bed area within the image may be set manually in advance or may be automatically detected by an image processing technology such as template matching.

In addition, the above description has exemplified lying in bed, rising on bed, chair sitting on bed end, leaving bed, and falling down as the motions detectable by the detection unit 150 but the detectable motions are not limited thereto. For example, the detectable motions may include other motions such as falling.

### (Motion set 122)

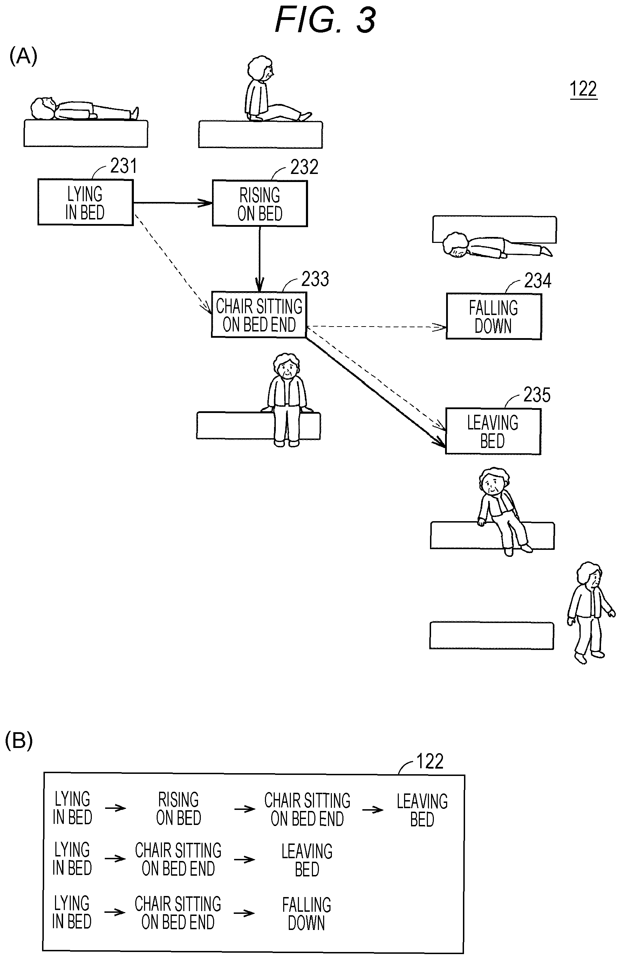

The motion set 122 will be described with reference to Fig. 3. Fig. 3 is a diagram illustrating the contents of the motion set 122 in the first embodiment.

A group of a series of motions arising in order of time series are defined in the motion set 122 as notification candidate motions that can be detected by the detection unit 150 (refer to Fig. 2). That is, the order of occurrence of motions is defined in the motion set 122. In the example in Fig. 3, the order of occurrence of lying in bed, rising on bed, chair sitting on bed end, falling down, and leaving bed is defined in the motion set 122.

In the following description, the order of occurrence of motions is indicated by an arrow "->". For example, when motions arise in the order of lying in bed, rising on bed, chair sitting on bed end, and leaving bed, a group of these motions are indicated as "lying in bed -> rising on bed -> chair sitting on bed end -> leaving bed".

As an example, during an interval from when the care receiver wakes up to when leaving the bed, a group of motions "lying in bed -> rising on bed -> chair sitting on bed end -> leaving bed" could arise. When the care receiver falls down from the bed, the care receiver falls down after moving the body once to a bed end and thus, the group of motions "lying in bed -> chair sitting on bed end -> falling" could arise. Meanwhile, the care receiver recover his/her balance in some cases even if he/she is about to fall down from the bed. In this case, a group of motions "lying in bed -> chair sitting on bed end -> leaving bed" could arise. Such groups of motions that could arise are defined in advance in the motion set 122.

Note that a correspondence relationship between motions is not limited to the example in Fig. 3. For example, falling that could arise after leaving the bed (that is, leaving bed -> falling) may be additionally defined in the motion set 122.

### (Selection Unit 160)

Referring again to Fig. 2, the selection unit 160 selects a notification target motion from among the notification candidate motions detected by the detection unit 150. Preferably, the selection unit 160 selects one motion from among the notification candidate motions as the notification target motion. At this time, the selection unit 160 changes the selection method for the notification target motion according to the correspondence relationship between the detected motion and the motion defined in the motion set 122. Details of selection processing by the selection unit 160 will be described later.

### (Output Unit 170)

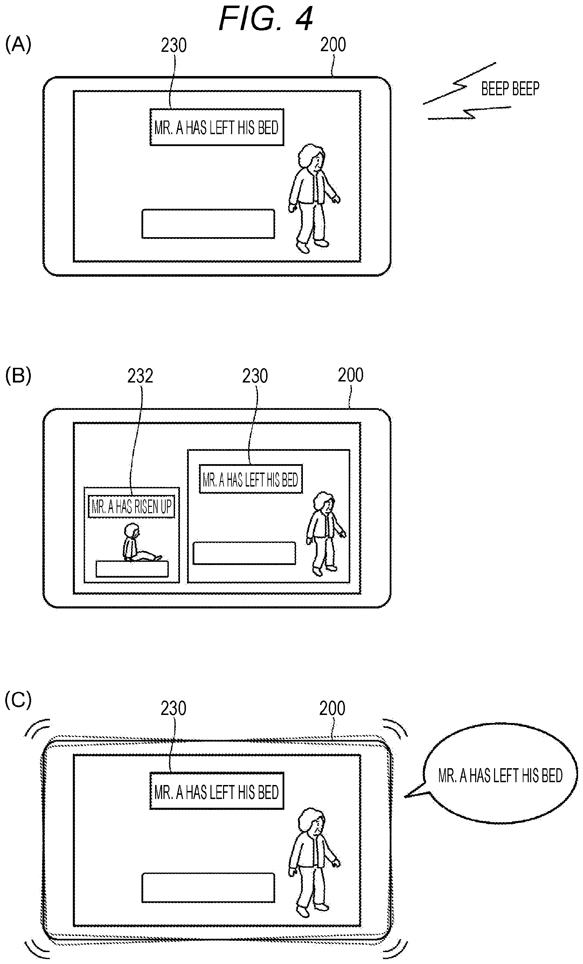

The output unit 170 (refer to Fig. 2) will be described with reference to Fig. 4. Fig. 4 is a diagram illustrating an output method for the detected motion. The output unit 170 is, for example, the mobile terminal 200 for the caregiver.

As illustrated in a specific example (A) in Fig. 4, the mobile terminal 200 displays a message 230 indicating the type of a detected motion and also issues a warning sound.

As another example, when there is a plurality of notification target motions, the mobile terminal 200 displays these motions in different display modes. As an example, the display mode can be changed depending on the display size. For example, as illustrated in a specific example (B) in Fig. 4, it is assumed that rising on bed and leaving bed are detected as the notification target motions. In a case where leaving bed is more important than the rising on bed, the output unit 170 makes the display size of the message 230 indicating leaving bed larger than the display size of a message 232 indicating rising on bed.

As illustrated in a specific example (C) in Fig. 4 as still another example, the mobile terminal 200 issues the notification target motion as a sound. Alternatively, the mobile terminal 200 may notify the caregiver of the notification target motion by oscillation (vibration).

Note that the output methods illustrated in the specific examples (A) to (C) in Fig. 4 may be appropriately combined or may be used alone. In addition, the output unit 170 is not limited to the mobile terminal 200. For example, the output unit 170 can also include a server for storing the notification target motion. An output result stored in the server is used, for example, to grasp a situation when the care receiver falls or falls down.

### [Specific Examples]

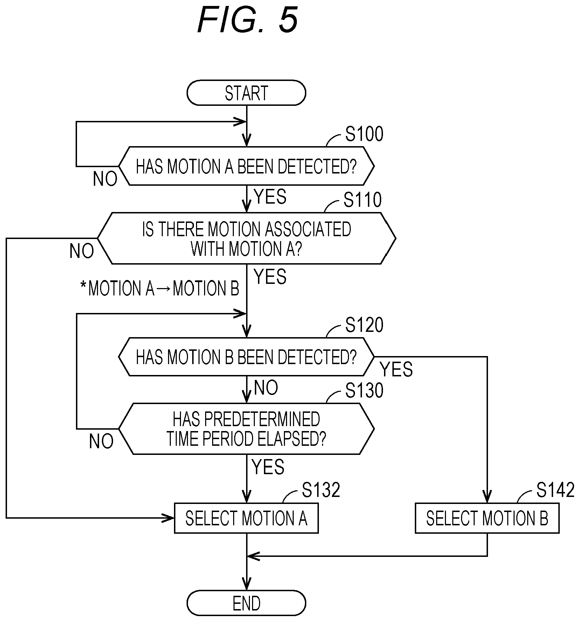

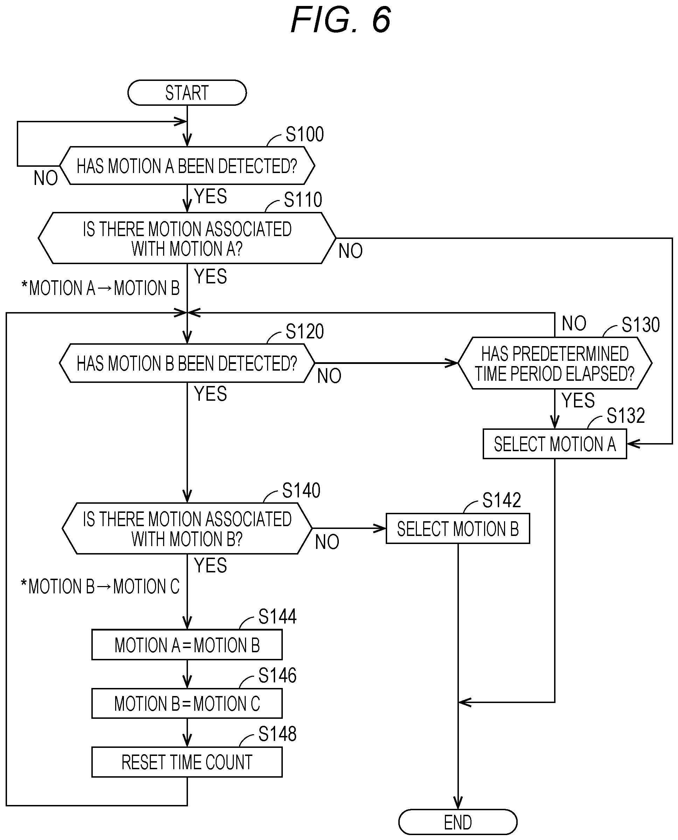

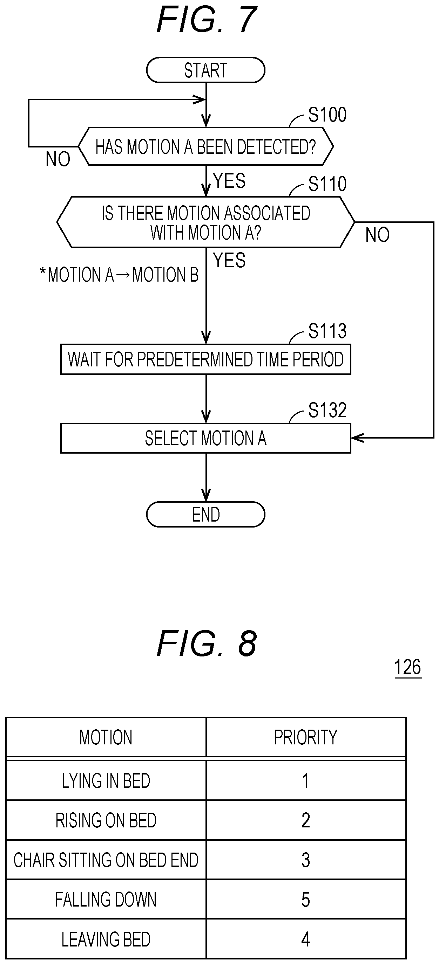

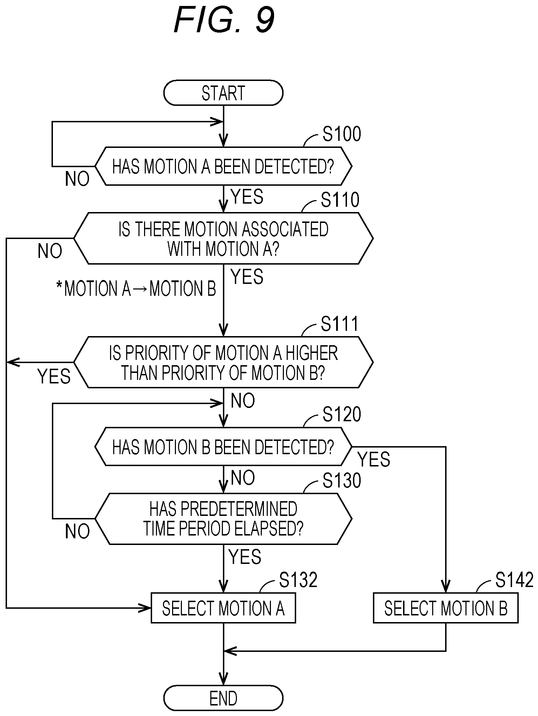

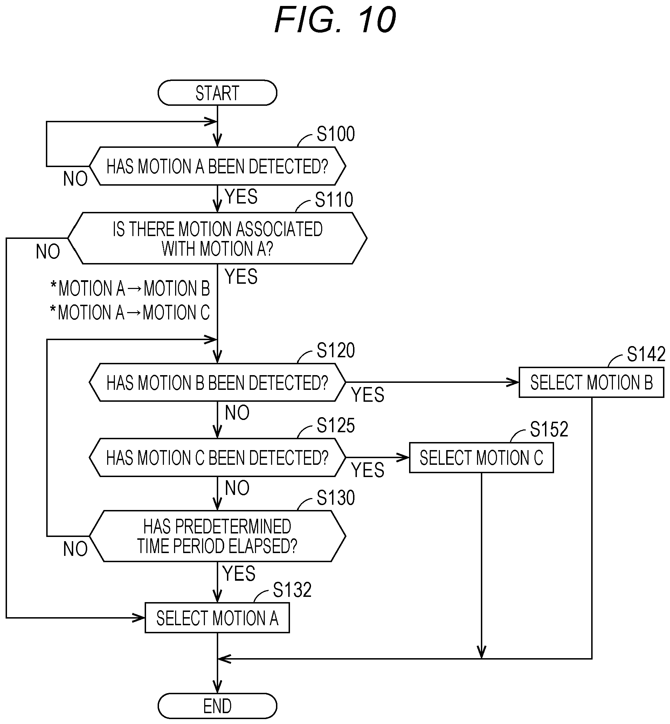

Details of the selection processing by the selection unit 160 (refer to Fig. 2) will be described with reference to Figs. 5 to 9. Fig. 5 is a flowchart illustrating a specific example 1-1 of the selection processing. Fig. 6 is a flowchart illustrating a specific example 1-2 of the selection processing. Fig. 7 is a flowchart illustrating a specific example 1-3 of the selection processing. Fig. 8 is a diagram illustrating the contents of the priority table 126. Fig. 9 is a flowchart illustrating a specific example 1-4 of the selection processing. Fig. 10 is a flowchart illustrating a specific example 1-5 of the selection processing.

The processing in Figs. 5 to 7, 9, and 10 is realized by the CPU 102 (refer to Fig. 2) functioning as the selection unit 160 executing a program. Alternatively, a part or all of the processing may be executed by a circuit element or other hardware.

### (Specific Example 1-1)

The specific example 1-1 of the selection processing by the selection unit 160 will be described with reference to Fig. 5. In the specific example 1-1, the selection unit 160 selects a last detected motion among the notification candidate motions detected during a predetermined time period as the notification target motion. The predetermined time period refers to a time period to wait to designate a notification candidate. That is, the selection unit 160 measures the predetermined time period after a first notification candidate motion is detected and assigns a motion detected within the predetermined time period as the notification candidate motion. In the following description, this predetermined time period is also referred to as "standby time period". The motion detection device 100 assigns the last detected motion among the notification candidates detected during the standby time period as the notification target, thereby being able to notify only the latest motion in a series of the motions. Consequently, the caregiver can accurately grasp the state of the care receiver.

As more specific processing, in step S 100, the selection unit 160 determines whether a motion A, which is a notification candidate, has been detected. When determining that the motion A has been detected (YES in step S100), the selection unit 160 switches the control to step S110. Otherwise (NO in step S100), the selection unit 160 executes the processing in step S100 again.

In step S110, the selection unit 160 refers to the motion set 122 (refer to Fig. 3) and determines whether there is a motion associated with the motion A. When determining that there is a motion associated with the motion A (YES in step S110), the selection unit 160 switches the control to step S120. Otherwise (NO in step S110), the selection unit 160 switches the control to step S132.

In step S120, the selection unit 160 determines whether a motion B associated with the motion A has been detected. When determining that the motion B has been detected (YES in step S120), the selection unit 160 switches the control to step S142. Otherwise (NO in step S 120), the selection unit 160 switches the control to step S130.

In step S130, the selection unit 160 determines whether the predetermined time period has elapsed since the motion A was detected. When determining that the predetermined time period has elapsed (YES in step S130), the selection unit 160 switches the control to step S132. Otherwise (NO in step S130), the selection unit 160 returns the control to step S120.

In step S132, the selection unit 160 selects the motion A as the notification target motion. In step S142, the selection unit 160 selects the motion B as the notification target motion.

The selection processing illustrated in Fig. 5 will be described by giving a further specific example. For example, it is assumed that a group of motions "chair sitting on bed end -> leaving bed" is defined in the motion set 122. In this case, the motion "chair sitting on bed end" acts as the motion A and the motion "leaving bed" acts as the motion B.

In a case where the motion "leaving bed" has been detected within the predetermined time period since the motion "chair sitting on bed end" was detected, the selection unit 160 selects the motion "leaving bed". In this case, the output unit 170 (refer to Fig. 2) notifies the caregiver of the care receiver leaving the bed. As an example, the output unit 170 serving as the mobile terminal 200 (refer to Fig. 1) issues an alarm sound, or activates a vibration function, or displays a message "Mr. A has left his bed" on the screen.

In a case where the motion "leaving bed" has not been detected within the predetermined time period since the motion "chair sitting on bed end" was detected, the selection unit 160 selects the motion "chair sitting on bed end". In this case, the output unit 170 notifies the caregiver of the care receiver chair sitting on the bed end. As an example, the output unit 170 serving as the mobile terminal 200 issues an alarm sound, or activates a vibration function, or displays a message "Mr. A has risen up" on the screen.

### (Specific Example 1-2)

In the specific example 1-1 described above, the notification candidate motions to be detected have been a group of time-series motions of two types. On the other hand, in the specific example 1-2, the notification candidate motions to be detected are a group of time-series motions of three or more types.

Hereinafter, selection processing according to the specific example 1-2 will be described with reference to Fig. 6. Note that processing in steps other than steps S120, S140, S146, and S148 illustrated in Fig. 6 is as described with reference to Fig. 5 and thus, description thereof will not be repeated.

In step S120, the selection unit 160 determines whether the motion B associated with the motion A has been detected. When determining that the motion B has been detected (YES in step S120), the selection unit 160 switches the control to step S140. Otherwise (NO in step S120), the selection unit 160 switches the control to step S130.

In step S140, the selection unit 160 refers to the motion set 122 (refer to Fig. 3) and determines whether there is a motion associated with the motion B. When determining that there is a motion associated with the motion B (YES in step S140), the selection unit 160 switches the control to step S144. Otherwise (NO in step S140), the selection unit 160 switches the control to step S142.

In step S144, the selection unit 160 replaces the motion A with the motion B. In step S146, the selection unit 160 replaces the motion B with a motion C. In step S148, the selection unit 160 resets a time count. As a result, the predetermined time period indicated in step S130 is reset.

The selection processing illustrated in Fig. 6 will be described by giving a further specific example. For example, it is assumed that a group of motions "rising on bed -> chair sitting on bed end -> leaving bed" is defined in the motion set 122. In a case where the motion "rising on bed" is detected, in an initial state, the motion "rising on bed" acts as the motion A, the motion "chair sitting on bed end" acts as the motion B, and the motion "leaving bed" acts as the motion C.

In a case where the motion "chair sitting on bed end" has been detected within the predetermined time period since the motion "rising on bed" was detected, the selection unit 160 replaces the motion A with the motion "chair sitting on bed end" in step S144. In step S146, the selection unit 160 replaces the motion B with the motion "leaving bed". Thereafter, in a case where "leaving bed" acting as the motion B has been detected within the predetermined time period since the motion "chair sitting on bed end" was detected, the selection unit 160 selects the motion "leaving bed" as the notification target motion (refer to step S142). In a case where "leaving bed" acting as the motion B has not been detected within the predetermined time period, the selection unit 160 selects, as the notification target motion, the motion "chair sitting on bed end" acting as the motion A (refer to step S132).

### (Specific Example 1-3)

The specific example 1-3 of the selection processing by the selection unit 160 will be described with reference to Fig. 7. In the specific example 1-3, the selection unit 160 selects a first detected motion among the notification candidates detected during the predetermined time period as the notification target motion. By notifying the first detected notification candidate, the time until the caregiver is notified is shortened. Consequently, the caregiver can promptly rush to the care receiver. In addition, the number of times of notifications to the caregiver is reduced and a burden on the caregiver is alleviated.

Note that processing in step S100 illustrated in Fig. 7 is as described with reference to Fig. 5 and thus, description thereof will not be repeated.

In step S110, the selection unit 160 refers to the motion set 122 and determines whether there is a motion associated with the motion A. When determining that there is a motion associated with the motion A (YES in step S110), the selection unit 160 switches the control to step S113. Otherwise (NO in step S110), the selection unit 160 switches the control to step S132.

In step S113, the selection unit 160 waits for the predetermined time period. As a result, the selection processing by the selection unit 160 is suspended for the predetermined time period. Even if the notification candidate motion is detected during this predetermined time period, the selection unit 160 ignores this motion.

In step S132, the selection unit 160 selects the motion A as the notification target motion.

The selection processing illustrated in Fig. 7 will be described by giving a further specific example. It is assumed that the motion "chair sitting on bed end" is detected in one aspect. In addition, it is assumed that a motion associated with the motion "chair sitting on bed end" is not defined in the motion set 122. In this case, the motion "chair sitting on bed end" acts as the motion A. The selection unit 160 selects the motion "chair sitting on bed end" as the notification target motion (refer to step S132).

In another aspect, it is assumed that the motion "chair sitting on bed end" is associated with the motion "leaving bed" in the motion set 122. In this case, the motion "chair sitting on bed end" acts as the motion A and the motion "leaving bed" acts as the motion B. After waiting for the predetermined time period in step S113 (refer to step S113), the selection unit 160 selects the motion "chair sitting on bed end" as the notification target motion in step S132 (refer to step S132).

For example, the output unit 170 serving as the mobile terminal 200 outputs "chair sitting on bed end" acting as the motion A that has been selected. As an example of the output method, the mobile terminal 200 issues an alarm sound, or activates a vibration function, or displays a message "Mr. A has risen up" on the screen.

### (Specific Example 1-4)

The specific example 1-4 of the selection processing by the selection unit 160 will be described with reference to Figs. 8 and 9. In the specific example 1-4, priorities are associated in advance with respective target motions to be detected. As illustrated in Fig. 8, a correspondence relationship between the type and the priority of a motion is defined in a priority table 126.

A higher priority is associated with a notification with higher necessity. In the example in Fig. 8, a priority "1" is associated with the motion "lying in bed". A priority "2" is associated with the motion "rising on bed". A priority "3" is associated with the motion "chair sitting on bed end". A priority "5" is associated with the motion "falling down". A priority "4" is associated with the motion "leaving bed".

The selection unit 160 selects a motion having a highest priority among the notification candidate motions as the notification target motion. By notifying a motion with a higher priority, the motion detection device 100 can preferentially notify a motion of the care receiver that needs care. In addition, the number of times of notifications to the caregiver is reduced and a burden on the caregiver is alleviated.

The selection processing of the specific example 1-4 will be described in more detail with reference to Fig. 9. Note that processing in steps other than step S111 illustrated in Fig. 9 is as described with reference to Fig. 5 and thus, description of these items of processing will not be repeated.

In step S111, the selection unit 160 determines whether the priority of the motion A is higher than the priority of the motion B. When determining that the priority of the motion A is higher than the priority of the motion B (YES in step S111), the selection unit 160 switches the control to step S132. Otherwise (NO in step S111), the selection unit 160 switches the control to step S120.

The selection processing illustrated in Fig. 9 will be described by giving a further specific example. For example, it is assumed that a group of motions "chair sitting on bed end -> leaving bed" is defined in the motion set 122. In this case, the motion "chair sitting on bed end" acts as the motion A and the motion "leaving bed" acts as the motion B. In the example in Fig. 8, the priority of the motion "chair sitting on bed end" (motion A) is "3", whereas the priority of the motion "leaving bed" (motion B) is "4". Since the priority of the motion B is higher than the priority of the motion A, the selection unit 160 switches the control from step S111 to step S120.

In a case where "leaving bed" acting as the motion B has not been detected within the predetermined time period (refer to steps S120 and S130), the selection unit 160 selects "chair sitting on bed end" acting as the motion A (refer to step S132). In a case where "leaving bed" acting as the motion B has been detected within the predetermined time period (refer to steps S120 and S130), the selection unit 160 selects "leaving bed" acting as the motion B (refer to step S142).

### (Specific Example 1-5)

The specific example 1-5 of the selection processing by the selection unit 160 will be described with reference to Fig. 10. Fig. 10 illustrates selection processing in a case where there is a plurality of candidates of a motion that could arise subsequently to a certain motion. Note that processing in steps S100 and S110 illustrated in Fig. 10 is as described with reference to Fig. 5 and thus, description thereof will not be repeated.

It is assumed that both of the motion B and the motion C are associated with the motion A in the motion set 122 (refer to Fig. 3). In this case, the selection unit 160 switches the control from step S110 to step S120.

In step S120, the selection unit 160 determines whether the motion B has been detected. When determining that the motion B has been detected (YES in step S120), the selection unit 160 switches the control to step S142. Otherwise (NO in step S120), the selection unit 160 switches the control to step S125.

In step S125, the selection unit 160 determines whether the motion C has been detected. When determining that the motion C has been detected (YES in step S125), the selection unit 160 switches the control to step S152. Otherwise (NO in step S125), the selection unit 160 switches the control to step S130.

In step S130, the selection unit 160 determines whether the predetermined time period has elapsed since the motion A was detected. When determining that the predetermined time period has elapsed (YES in step S130), the selection unit 160 switches the control to step S132. Otherwise (NO in step S130), the selection unit 160 returns the control to step S120.

In step S132, the selection unit 160 selects the motion A as the notification target motion. In step S142, the selection unit 160 selects the motion B as the notification target motion. In step S152, the selection unit 160 selects the motion C as the notification target motion.

The selection processing illustrated in Fig. 10 will be described by giving a further specific example. For example, it is assumed that a group of motions "chair sitting on bed end -> leaving bed" and a group of motions "chair sitting on bed end -> falling down" are defined in the motion set 122. In this case, the motion "chair sitting on bed end" acts as the motion A, the motion "leaving bed" acts as the motion B, and the motion "falling down" acts as the motion C.

In a case where "leaving bed" acting as the motion B and "falling down" acting as the motion C have not been detected within the predetermined time period since "chair sitting on bed end" acting as the motion A was detected, the selection unit 160 selects, as the notification target motion, "chair sitting on bed end" acting as the motion A (refer to step S132). In a case where "leaving bed" acting as the motion B has been detected within this predetermined time period, the selection unit 160 selects the motion "leaving bed" as the notification target motion (refer to step S142). In a case where "falling down" acting as the motion C has been detected within this predetermined time period, the selection unit 160 selects the motion "falling down" as the notification target motion (refer to step S152).

In a case where the motion "leaving bed" is selected by the selection unit 160, the output unit 170 (refer to Fig. 2) notifies the caregiver of the care receiver leaving the bed. As an example, the output unit 170 serving as the mobile terminal 200 (refer to Fig. 1) issues an alarm sound, or activates a vibration function, or displays a message "Mr. A has left his bed".

In a case where the motion "falling down" is selected by the selection unit 160, the output unit 170 notifies the caregiver of the care receiver falling down. As an example, the output unit 170 serving as the mobile terminal 200 issues an alarm sound, or activates a vibration function, or displays a message "Mr. A has dropped out of the bed. Please check the situation" on the screen.

In a case where the motion "chair sitting on bed end" is selected by the selection unit 160, the output unit 170 notifies the caregiver of the care receiver chair sitting on the bed end. As an example, the output unit 170 serving as the mobile terminal 200 issues an alarm sound, or activates a vibration function, or displays a message "Mr. A has risen up" on the screen.

### [Hardware Configuration of Motion Detection System 300]

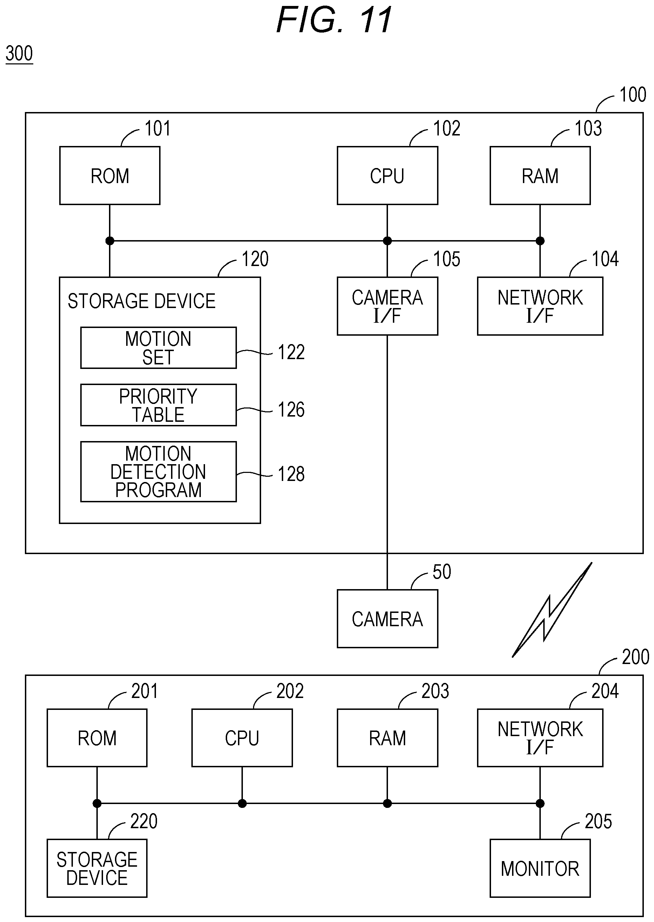

An example of a hardware configuration of the motion detection system 300 will be described with reference to Fig. 11. Fig. 11 is a block diagram illustrating a main hardware configuration of the motion detection system 300. As illustrated in Fig. 11, the motion detection system 300 includes the motion detection device 100 and the mobile terminal 200. The motion detection device 100 and the mobile terminal 200 are connected to each other via the network. Hereinafter, the hardware configuration of the motion detection device 100 and the hardware configuration of the mobile terminal 200 will be described in order.

### (Hardware Configuration of Motion Detection Device 100)

As illustrated in Fig. 11, the motion detection device 100 includes a read only memory (ROM) 101, the CPU 102, a random access memory (RAM) 103, a network interface (I/F) 104, a camera I/F 105, and the storage device 120.

An operating system, a motion detection program 128 according to the present embodiment, and so on are saved in the ROM 101. The CPU 102 executes various programs such as the operating system and the motion detection program 128 to control the operation of the motion detection device 100. The RAM 103 functions as a working memory and various items of data necessary for executing the motion detection program 128 are temporarily saved therein.

A communication apparatus such as an antenna or a network interface card (NIC) is connected to the network I/F 104. The motion detection device 100 transmits and receives data to and from another communication terminal via this communication apparatus. Another communication terminal includes, for example, the mobile terminal 200, a server, and other terminals. The motion detection device 100 may be configured to be able to download the motion detection program 128 from a server via the network.

The camera I/F 105 is an interface for connecting the camera 50 and the motion detection device 100 in a wired or wireless manner. The motion detection device 100 acquires an image from the camera 50 via the camera I/F 105. The camera 50 includes, for example, a network camera or another imaging device capable of capturing a subject. The camera 50 may be configured integrally with the motion detection device 100 or may be configured separately from the motion detection device 100 as illustrated in Fig. 11.

The storage device 120 is, for example, a storage medium such as a hard disk or an external storage device. As an example, the motion set 122, the priority table 126, the motion detection program 128 according to the present embodiment, and so on are saved in the storage device 120.

Note that the motion detection program 128 may be provided as a part of an arbitrary program by being embedded therein instead of being provided as an independent program. In this case, the processing according to the present embodiment is realized in cooperation with the arbitrary program. Even such a program that does not include some of modules does not depart from the gist of the motion detection device 100 according to the present embodiment. Furthermore, some or all of the functions provided by the motion detection program 128 according to the present embodiment may be realized by dedicated hardware. Additionally, some or all of the functions provided by the motion detection program 128 may be realized by the motion detection device 100 and the mobile terminal 200 cooperating with each other. Furthermore, the motion detection device 100 may be configured in a form such as a so-called cloud service in which at least one server realizes the processing according to the present embodiment.

### (Hardware Configuration of Mobile Terminal 200)

The hardware configuration of mobile terminal 200 will be described with continued reference to Fig. 11. The mobile terminal 200 is, for example, a smartphone, a tablet terminal, and another terminal that a caregiver can carry. As illustrated in Fig. 11, the mobile terminal 200 includes a ROM 201, a CPU 202, a RAM 203, a network I/F 204, a display 205, and a storage device 220.

An operating system, a control program executed by the mobile terminal 200, and so on are saved in the ROM 201. The CPU 202 executes various programs such as the operating system and the control program for the mobile terminal 200 to control the operation of the mobile terminal 200. The RAM 203 functions as a working memory and various items of data necessary for executing the programs are temporarily saved therein.

A communication apparatus such as an antenna or a network interface card (NIC) is connected to the network I/F 204. The mobile terminal 200 transmits and receives data to and from another communication terminal via this communication apparatus. Another communication terminal includes, for example, the motion detection device 100 and other terminals.

The display 205 is, for example, a liquid crystal display, an organic electro luminescence (EL) display, or another display apparatus. The display 205 may be configured as a touch panel. The display 205 displays that the notification target motion of the care receiver has been detected in a case where this motion has been detected.

The storage device 220 includes, for example, a storage medium such as an embedded MultiMediaCard (eMMC). The eMMC includes a NAND flash memory and a control circuit. A program for realizing various items of processing according to the present embodiment and so on are saved in the storage device 220.

### [Brief Review]

As described above, when a group of a series of motions that could arise in order of time series is detected during the predetermined time period as the notification candidate motions, the motion detection system 300 selects a notification target motion from among the notification candidate motions. As a result, it is possible to prevent the detected motions from being notified each time and the number of times of notifications to the caregiver is reduced. Since the notification content is simplified by reducing the number of times of notifications, the caregiver can easily grasp the notification content. In addition, since the caregiver does not need to deal with unnecessary notifications or low emergency notifications, a burden on the caregiver is alleviated.

### <Second Embodiment>

### [Overview]

In the first embodiment, a time period to wait to detect the notification candidate (that is, the "standby time period") has been constant. On the other hand, a motion detection system 300 according to a second embodiment changes this standby time period according to the notification candidate motion to be detected.

### [Processing by Motion Detection System 300]

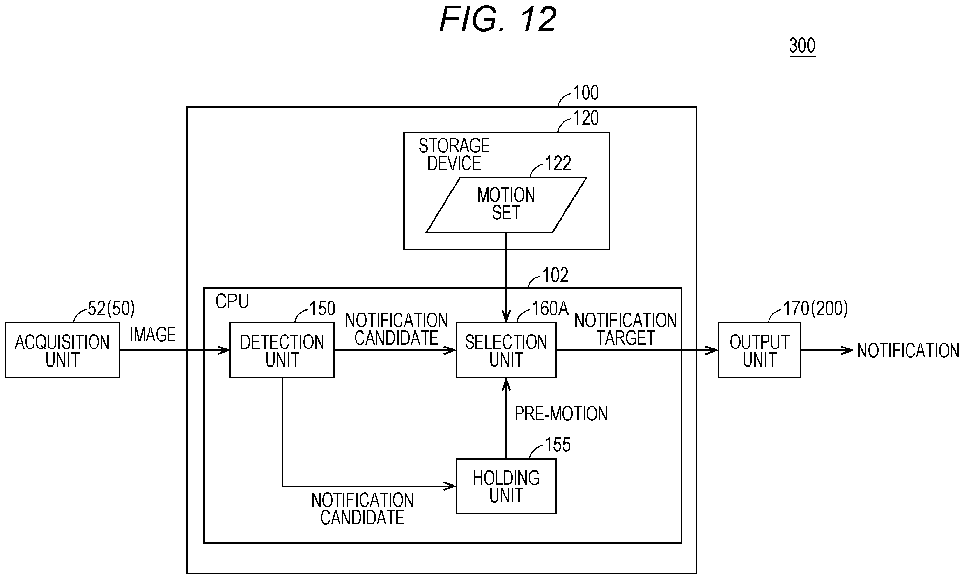

Motion detection processing by the motion detection system 300 according to the second embodiment will be described with reference to Fig. 12. Fig. 12 is a block diagram illustrating a hardware configuration and a functional configuration of the motion detection system 300 according to the second embodiment. As illustrated in Fig. 12, the motion detection system 300 is provided with an acquisition unit 52, a motion detection device 100, and an output unit 170. The motion detection device 100 includes a CPU 102 and a storage device 120 as a hardware configuration. The CPU 102 has a detection unit 150, a holding unit 155, and a selection unit 160A as a functional configuration. Hereinafter, the holding unit 155 and the selection unit 160A will be described in order. Since other configurations are as described above, description of these configurations will not be repeated.

The holding unit 155 holds the notification candidate motion detected by the detection unit 150 for a given period as history information. This history information may be held in a cache memory of the CPU 102 or may be held in the storage device 120. In addition, the holding unit 155 may hold only one notification candidate as the history information or may hold the notification candidates for past several times as the history information.

When the notification candidate motion is detected, the selection unit 160A changes the standby time period for detecting the notification candidate according to a motion detected previously to the above motion (hereinafter also referred to as "pre-motion"). Details of the selection unit 160A will be described later.

### [Specific Example 2]

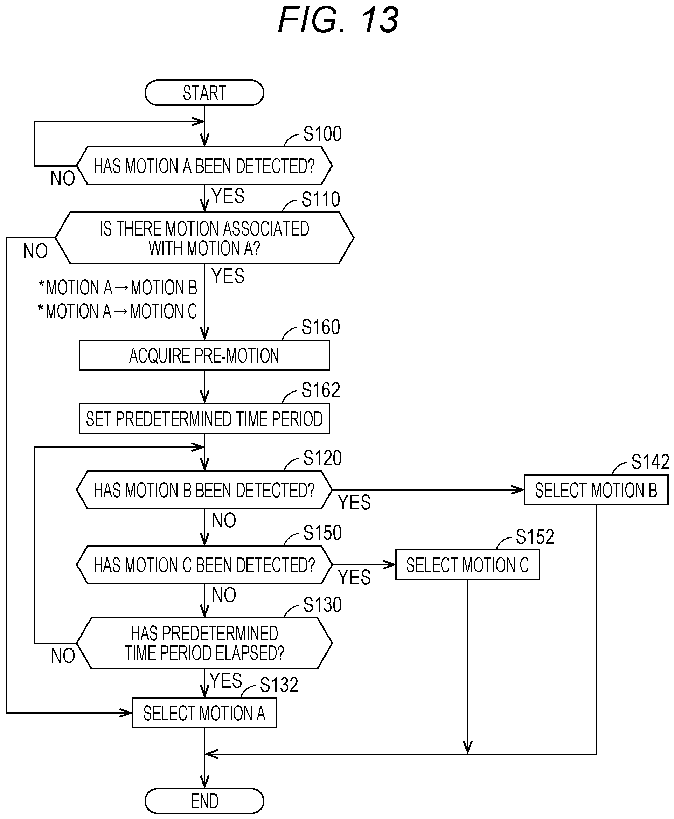

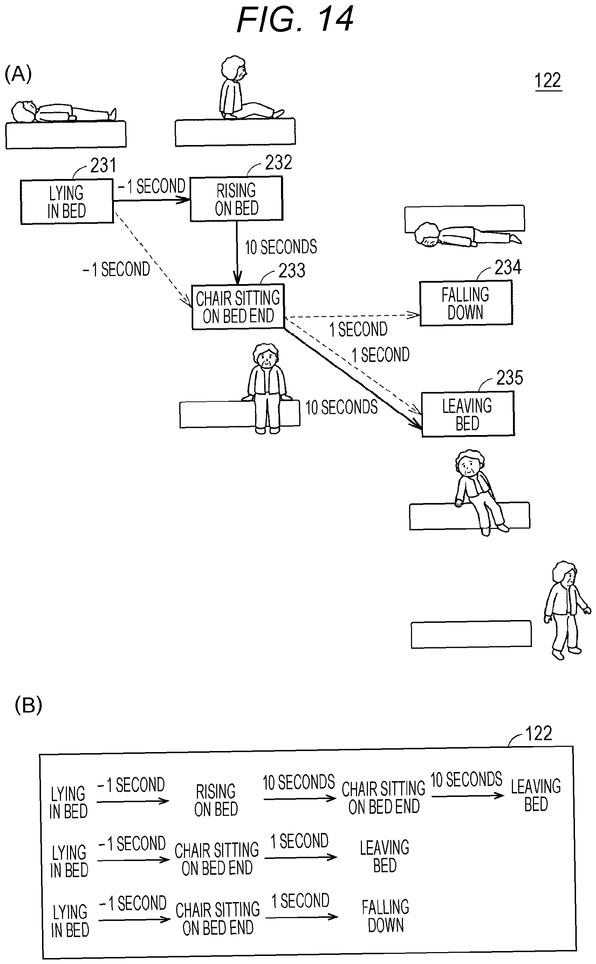

The selection processing by the selection unit 160A illustrated in Fig. 12 will be described in more detail with reference to Figs. 13 and 14. Fig. 13 is a flowchart illustrating a specific example 2 of the selection processing. Fig. 14 is a diagram illustrating the contents of a motion set 122 in the second embodiment. Note that processing in steps other than steps S160 and S162 illustrated in Fig. 13 is as described with reference to Fig. 10 and thus, description thereof will not be repeated.

In step S160, the selection unit 160A acquires a motion detected immediately before the motion A (that is, a pre-motion) from the holding unit 155 (refer to Fig. 12).

In step S162, the selection unit 160A changes the standby time period for detecting the notification candidate according to the acquired pre-motion. The standby time period is associated in advance with each target motion in the groups to be detected.

The selection processing illustrated in Fig. 13 will be described in more detail with reference to Fig. 14. For example, it is assumed that a group of motions "lying in bed -> rising on bed -> chair sitting on bed end -> leaving bed", a group of motions "lying in bed -> chair sitting on bed end -> leaving bed", and a group of motions "lying in bed -> chair sitting on bed end -> falling down" are defined in the motion set 122.

That is, the motion "lying in bed" or the motion "rising on bed" could arise before the motion "chair sitting on bed end". When the motion "chair sitting on bed end" is detected, the selection unit 160A changes the standby time period according to whether the pre-motion detected before the motion "chair sitting on bed end" is the motion "lying in bed" or the motion "rising on bed".

For example, it is assumed that the motion "lying in bed" has been detected immediately before the motion "chair sitting on bed end". The selection unit 160A refers to the motion set 122 to acquire a group of motions including the motion "chair sitting on bed end" and having "lying in bed" as the pre-motion. That is, the selection unit 160A acquires the group of motions "lying in bed -> chair sitting on bed end -> leaving bed" and the group of motions "lying in bed -> chair sitting on bed end -> falling down".

In the example in Fig. 14, a standby time period "1 second" is associated with "chair sitting on bed end -> leaving bed" in the group of motions "lying in bed -> chair sitting on bed end -> leaving bed". In addition, a standby time period "1 second" is associated with "chair sitting on bed end -> falling down" in the group of motions "lying in bed -> chair sitting on bed end -> falling down". Therefore, the selection unit 160A changes the standby time period to "1 second". If different standby time periods are associated with "chair sitting on bed end -> falling down" and "chair sitting on bed end -> leaving bed", the selection unit 160A updates a current standby time period with a shorter standby time period.

Note that, when a group of motions associated with a standby time period "-1 second" is detected, the motion detection system 300 does not notify a motion detected earlier among this group of motions.

### [Brief Review]

As described above, the motion detection system 300 according to the present embodiment changes the standby time period for detecting the notification candidate according to a motion previously detected. As a result, the motion detection system 300 can instantaneously notify a high emergency motion such as falling down.

### <Third Embodiment>

### [Overview]

In the first embodiment, the standby time period for detecting the notification candidate has been constant irrespective of an output means for the notification target motion. On the other hand, in a third embodiment, the standby time period is different for each output means.

### [Processing by Motion Detection System 300]

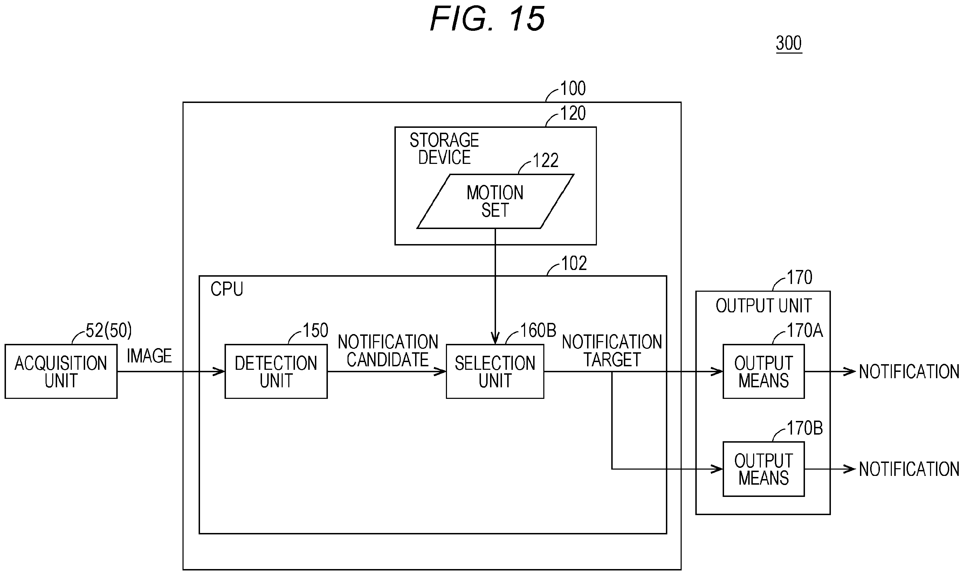

Motion detection processing by a motion detection system 300 according to the third embodiment will be described with reference to Fig. 15. Fig. 15 is a block diagram illustrating a hardware configuration and a functional configuration of the motion detection system 300 according to the third embodiment. As illustrated in Fig. 15, the motion detection system 300 is provided with an acquisition unit 52, a motion detection device 100, and an output unit 170. The motion detection device 100 includes a CPU 102 and a storage device 120 as a hardware configuration. The CPU 102 has a detection unit 150, a holding unit 155, and a selection unit 160B as a functional configuration. Hereinafter, the selection unit 160B and the output unit 170 will be described. Since other configurations are as described above, description of these configurations will not be repeated.

The output unit 170 includes a plurality of output means to output the notification target motion in different methods. For example, the output unit 170 includes an output means 170A and an output means 170B. The selection unit 160B selects the notification candidate motion using the standby time period different for each output means. That is, the selection unit 160B performs the selection processing for the notification target motion with respect to each of the output means.

In one aspect, the output means 170A is a mobile terminal 200 for notifying the caregiver of the notification target motion. The output means 170B is a server (not illustrated) for saving the notification target motion in a built-in memory. The selection unit 160B selects the notification candidate motion for the output means 170A at every first predetermined time period and selects the notification candidate motion for the output means 170B at every second predetermined time period shorter than the first predetermined time period.

In another aspect, the output means 170A is a mobile terminal 200 for notifying the caregiver of the notification target motion. This mobile terminal 200 is a terminal prepared for use by the caregiver supposed to rush to the care receiver. The output means 170B is a mobile terminal (not illustrated) for notifying an administrator of the motion detection system 300 of the notification target motion. This mobile terminal is a terminal prepared for use by the administrator not supposed to rush to the care receiver. Alternatively, the output means 170B may be a fixed terminal disposed in a central monitoring center or the like that manages each nursing facility. The selection unit 160B selects the notification candidate motion for the output means 170A at every first predetermined time period and selects the notification candidate motion for the output means 170B at every second predetermined time period shorter than the first predetermined time period.

### [Specific Example 3]

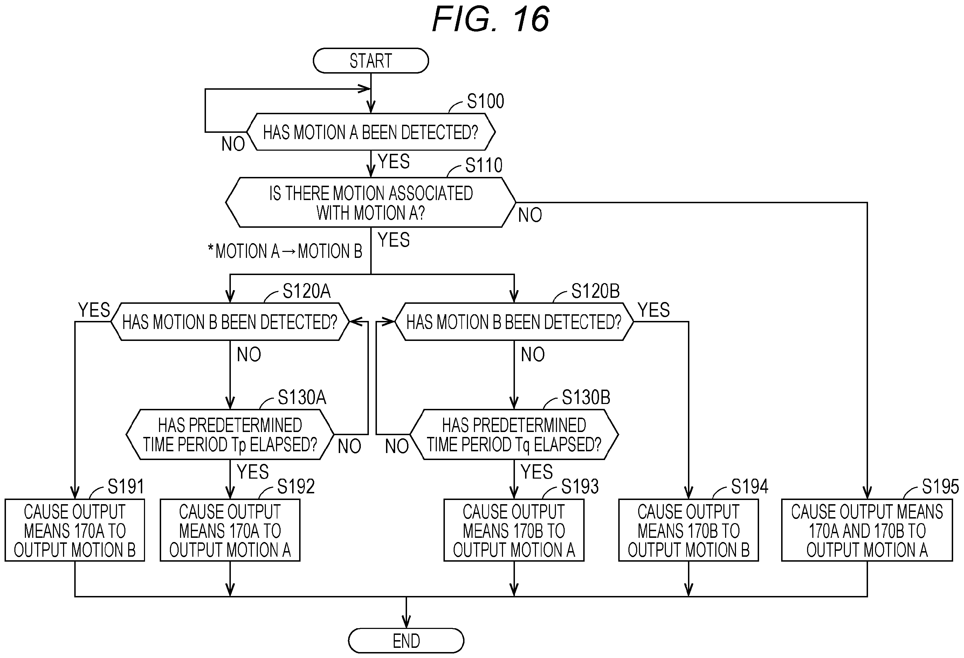

The selection processing by the selection unit 160B illustrated in Fig. 15 will be described in more detail with reference to Fig. 16. Fig. 16 is a flowchart illustrating a specific example 3 of the selection processing.

In step S100, the selection unit 160B determines whether the motion A defined in the motion set 122 (refer to Fig. 3) has been detected. When determining that the motion A has been detected (YES in step S100), the selection unit 160B switches the control to step S110. Otherwise (NO in step S100), the selection unit 160B executes the processing in step S100 again.

In step S110, the selection unit 160B refers to the motion set 122 and determines whether there is a motion associated with the motion A. When determining that there is a motion associated with the motion A (YES in step S110), the selection unit 160B switches the control to steps S120A and S120B. Otherwise (NO in step S110), the selection unit 160B switches the control to step S195.

The processing in steps S120A and S120B and thereafter are executed in parallel. In step S120A, the selection unit 160B determines whether the motion B associated with the motion A has been detected. When determining that the motion B has been detected (YES in step S120A), the selection unit 160B switches the control to step S191. Otherwise (NO in step S120A), the selection unit 160B switches the control to step S130A.

In step S120B, the selection unit 160B determines whether the motion B associated with the motion A has been detected. When determining that the motion B has been detected (YES in step S120B), the selection unit 160B switches the control to step S194. Otherwise (NO in step S120B), the selection unit 160B switches the control to step S130B.

In step S130A, the selection unit 160B determines whether a predetermined time period Tp (standby time period) has elapsed since the motion A was detected. The predetermined time period Tp is, for example, 10 seconds. When determining that the predetermined time period Tp has elapsed (YES in step S130A), the selection unit 160B switches the control to step S192. Otherwise (NO in step S130A), the selection unit 160B returns the control to step S120A.

In step S130B, the selection unit 160B determines whether a predetermined time period Tq has elapsed since the motion A was detected. The predetermined time period Tq is different from the predetermined time period Tp. The predetermined time period Tq is, for example, 0.1 seconds. When determining that the predetermined time period Tq has elapsed (YES in step S130B), the selection unit 160B switches the control to step S193. Otherwise (NO in step S130B), the selection unit 160B returns the control to step S120B.

In step S191, the selection unit 160B causes the output means 170A to output the motion B. In step S192, the selection unit 160B causes the output means 170A to output the motion A. In step S193, the selection unit 160B causes the output means 170B to output the motion A. In step S194, the selection unit 160B causes the output means 170B to output the motion B. In step S195, the selection unit 160B causes the output means 170A and 170B to output the motion A.

The selection processing illustrated in Fig. 16 will be described by giving a further specific example. In one aspect, it is assumed that the motion "chair sitting on bed end" is defined in the motion set 122 and no motion is associated with this "chair sitting on bed end". In this case, the selection unit 160B selects the motion "chair sitting on bed end" and also causes each of the output means 170A and 170B to output the motion "chair sitting on bed end" (refer to step S195).

In another aspect, it is assumed that a group of motions "chair sitting on bed end -> leaving bed" is defined in the motion set 122. In this case, the motion "chair sitting on bed end" acts as the motion A and the motion "leaving bed" acts as the motion B. When "leaving bed" acting as the motion B has been detected within the predetermined time period Tp, the selection unit 160B selects "leaving bed" acting as the motion B and also causes the output means 170A to output the motion "leaving bed" (refer to step S191). When "leaving bed" acting as the motion B has not been detected within the predetermined time period Tp, the selection unit 160B selects "chair sitting on bed end" acting as the motion A and also causes the output means 170A to output the motion "chair sitting on bed end" (refer to step S192).

When "leaving bed" acting as the motion B has been detected within the predetermined time period Tq, the selection unit 160B selects "leaving bed" acting as the motion B and also causes the output means 170B to output the motion "leaving bed" (refer to step S194). When "leaving bed" acting as the motion B has not been detected within the predetermined time period Tq, the selection unit 160B selects "chair sitting on bed end" acting as the motion A and also causes the output means 170B to output the motion "chair sitting on bed end" (refer to step S193).

When outputting the motion "leaving bed", the output means 170A serving as the mobile terminal 200 issues an alarm sound, or activates a vibration function, or displays a message "Mr. A has left his bed" on the screen.

When outputting the motion "chair sitting on bed end", the output means 170A serving as the mobile terminal 200 issues an alarm sound, or activates a vibration function, or displays a message "Mr. A has risen up" on the screen.

When outputting the motion "leaving bed", the output means 170B serving as a server (not illustrated) displays a message "Mr. A has left his bed" on the screen.

When outputting the motion "chair sitting on bed end", the output means 170B serving as a server displays a message "Mr. A has risen up" on the screen. Alternatively, the output means 170B serving as a server associates the type of the notification target motion with the time at which this motion was detected and then writes a correspondence relationship therebetween in the history information.

### [Brief Review]

As described above, the motion detection system 300 according to the present embodiment designates the notification candidate using the standby time period different for each output means and executes the selection processing for the notification target motion with respect to each of the output means. Since the standby time period can be set in line with the intended use of the output means, a motion selection result suitable for each output means can be output.

### <Fourth Embodiment>

In the third embodiment, the standby time period for detecting the notification candidate motion has been different for each output means. On the other hand, in a fourth embodiment, the standby time period is different for each care receiver to be monitored. This standby time period is set in advance for each care receiver in line with the health condition and physical ability of the care receiver. As a result, the motion detection system 300 can accurately detect the motion of the care receiver without being influenced by the health condition and the physical ability of the care receiver (for example, a motion speed).

It should be considered that the embodiments disclosed this time are examples in all respects and not restrictive. The scope of the present invention is defined not by the description above but by the claims and it is intended that all modifications within the meaning and scope of the claims and the equivalents thereof are included.

### Reference Signs List

10 Care receiver
11 Caregiver
20 Bed
50 Camera
52 Acquisition unit
100 Motion detection device
101, 201 ROM
102, 202 CPU
103, 203 RAM
104, 204 Network I/F
105 Camera I/F
120, 220 Storage device
122 Motion set
126 Priority table
128 Motion detection program
150 Detection unit
155 Holding unit
160, 160A, 160B Selection unit
170 Output unit
170A, 170B Output means
200 Mobile terminal
205 Display
230, 232 Message
300 Motion detection system

## Claims

1. A motion detection system comprising:
an acquisition unit for acquiring motion data indicating a motion of a person;
a detection unit for detecting a predetermined motion of the person using the motion data;
a selection unit for, when a plurality of types of output candidate motions is detected by the detection unit during a predetermined time period, selecting an output target motion from among the output candidate motions such that types of the output candidate motions are made fewer than the plurality of types; and
an output unit for outputting the output target motion selected by the selection unit.

2. The motion detection system according to claim 1, wherein the plurality of types of the output candidate motions includes a group of motions that could arise during an interval from when the person is sleeping in a bed to when leaving the bed.

3. The motion detection system according to claim 1 or 2, wherein the plurality of types of the output candidate motions is a group of a series of motions that could arise in order of time series.

4. The motion detection system according to claim 3, wherein the selection unit selects a last detected motion among the output candidate motions as the output target motion.

5. The motion detection system according to claim 3, wherein the selection unit selects a first detected motion among the output candidate motions as the output target motion.

6. The motion detection system according to claim 3, wherein
a priority is associated in advance with each of target motions to be detected by the detection unit, and
the selection unit selects a motion having a highest priority among the output candidate motions as the output target motion.

7. The motion detection system according to any one of claims 3 to 6, wherein
the group of the series of the motions includes a first motion, a second motion, and a third motion,
the first motion and the second motion are motions that could arise before the third motion, and
when the third motion is detected, the selection unit changes the predetermined time period according to whether a motion detected before the third motion is the first motion or the second motion.

8. The motion detection system according to any one of claims 3 to 7, wherein
the output unit outputs the output target motions by a plurality of different output means, and
the selection unit selects one of the output candidate motions using the predetermined time period different for each of the plurality of output means.

9. The motion detection system according to claim 8, wherein
the output unit includes, as the plurality of output means:
a first output means for notifying a caregiver for the person of the output target motion; and
a second output means for saving the output target motion to a memory, and
the selection unit:
selects one of the output candidate motions for the first output means at every first predetermined time period; and
selects one of the output candidate motions for the second output means at every second predetermined time period shorter than the first predetermined time period.

10. The motion detection system according to claim 8, wherein
the output unit includes, as the plurality of output means:
a first output means for notifying a caregiver for the person of the output target motion; and
a second output means for notifying an administrator of the motion detection system of the output target motion, and
the selection unit:
selects one of the output candidate motions for the first output means at every first predetermined time period; and
selects one of the output candidate motions for the second output means at every second predetermined time period shorter than the first predetermined time period.

11. A motion detection device comprising:
an acquisition unit for acquiring motion data indicating a motion of a person;
a detection unit for detecting a predetermined motion of the person using the motion data;
a selection unit for, when a plurality of types of output candidate motions is detected by the detection unit during a predetermined time period, selecting an output target motion from among the output candidate motions such that types of the output candidate motions are made fewer than the plurality of types; and
an output unit for outputting the output target motion selected by the selection unit.

12. A motion detection method comprising:
a step of acquiring motion data indicating a motion of a person;
a step of detecting a predetermined motion of the person using the motion data;
a step of, when a plurality of types of output candidate motions is detected in the step of detecting during a predetermined time period, selecting an output target motion from among the output candidate motions such that types of the output candidate motions are made fewer than the plurality of types; and
a step of outputting the output target motion selected in the step of selecting.

13. A motion detection program that causes a computer to execute:
a step of acquiring motion data indicating a motion of a person;
a step of detecting a predetermined motion of the person using the motion data;
a step of, when a plurality of types of output candidate motions is detected in the step of detecting during a predetermined time period, selecting an output target motion from among the output candidate motions such that types of the output candidate motions are made fewer than the plurality of types; and
a step of outputting the output target motion selected in the step of selecting.
